(19) Europäisches Patentamt
European Patent Office
Office européen des brevets

(11) Publication number : 0 014 515 B2

(12) NEW EUROPEAN PATENT SPECIFICATION

(45) Date of publication of the new patent specification : 13.03.91 Bulletin 91/11

(51) Int. Cl.$^5$ : C08F 265/06, C08F 291/00, A61K 6/00

(21) Application number : 80300030.6

(22) Date of filing : 04.01.80

# (54) Hardenable compositions, process to make a shaped article therefrom and dental appliance comprising it

The file contains technical information submitted after the application was filed and not included in this specification

(30) Priority : 01.02.79 US 8507

(43) Date of publication of application : 20.08.80 Bulletin 80/17

(45) Publication of the grant of the patent : 14.03.84 Bulletin 84/11

(45) Mention of the opposition decision : 13.03.91 Bulletin 91/11

(84) Designated Contracting States : AT BE CH DE FR GB IT LU NL SE

(56) References cited :
DE-C- 2 403 211
FR-A- 2 091 046
GB-A- 569 975
GB-A- 801 824
GB-A- 983 826
GB-A- 1 254 226

(56) References cited :
US-A- 3 210 212
US-A- 3 534 122
US-A- 3 914 341
US-A- 3 961 379
"Polymer Blends and Composites", 2nd edition, 1977, Heyden, New York and London, p. 53-54 and 237-256, article by John A. Manson and Leslie H. Sperling
Ullmann, Encyclopedia, 5th edition, 1987, Vol.A 8, page 24

(73) Proprietor : DENTSPLY INTERNATIONAL, INC.
570 West College Avenue P.O. Box 872
York Pennsylvania 17405 (US)

(72) Inventor : Roemer, Frederick Donald
479 S. Pleasant Avenue
Dallastown Pennsylvania (US)
Inventor : Tateosian, Louis Hagop
209 Reynolds Mill Road
York Pennsylvania (US)

(74) Representative : Wächtershäuser, Günter, Dr.
Tal 29
W-8000 München 2 (DE)

EP 0 014 515 B2

## Description

Process for producing a hard, water insensitive shaped dental appliance having a high grind resistance

This invention provides a process for producing shaped dental appliances.

Artificial teeth should exhibit certain physical and physiochemical characteristics to be suitable for use. They should be hard and resistant to chipping, durable, and stable to solvents, water, and heat. In addition, they should be of an aesthetically acceptable color, i.e., close to that of natural teeth, or be amenable to artificial coloration. The teeth should not cause excessive wear to opposing natural or artificial teeth, should not wear out of occlusion, and should be capable of being bonded firmly to supportive structures. They should also be amenable to ordinary means of physical shaping, grinding, and polishing, so as to minimize production costs.

Various metals and ceramics have been traditionally used for the formation of artificial teeth and other dental appliances. These, however, possess certain inherent deficiencies which lessen their desirability in dental applications. Thus, the metallic color of gold, amalgam, and other metallic species serves as an aesthetic detraction to the wearer of appliances made therefrom. In addition, the high cost of most noble metals from which many such appliances are commonly made leads to a cost consideration whenever their use is contemplated. Ceramic materials, another common alternative, are often difficult to form into acceptable shapes, and may tend to evidence abrasive and aesthetically unpleasant subsurfaces upon the physical wearing-away of surface layers. Such materials are also difficult to polish satisfactorily. These reasons together with factors related to cost, to consumer preference, to the technical skills of dental practitioners, and to convenience have motivated a search for alternative compositions suitable for the construction of dental appliances, especially artificial teeth.

Of the presently available organic compositions used for the construction of artificial teeth, most are composed of acrylics, often crosslinked by polyfunctional moieties. While such compositions are now commonly in use, they nonetheless possess certain drawbacks. In general, currently available acrylic compositions are only poorly resistant to wearing and grinding and may stain easily. Moreover, their mechanical workability is poor and they tend to melt and smear when ground. Their resistance to heat and to solvents is often poor, and in some cases, they are hydrolytically unstable.

Accordingly, it is the object of this invention to provide a process for producing shaped dental appliances having improved workability and superior physical and aesthetic characteristics.

It is to be understood that the term "bisphenol-A" is commonly utilized in the art to indicate the chemical compound 2,2-bis(4-hydroxyphenyl)propane. It is also to be understood that the term "bisGMA" is commonly used to indicate the chemical compound 2,2-bis[4-(2-hydroxy-3-methacryloxypropoxy)phenyl]propane, otherwise referred to as "digycidyl methacrylate of bisphenol-A".

U.S. patent 3,084,436 to Laudry discloses soft dental materials manufactured from mixtures of methacrylate monomers. Monofunctional esters together with vinyl acetate or vinyl stearate are crosslinked with polyfunctional esters of acrylic or methacrylic acid. The resulting product is disclosed as being three dimensionally crosslinked.

The preparation of graft copolymers is disclosed by U.S. patent 3,087,875 to Graham et al. Alkyl methacrylate and analogous polymers are dissolved in monomers such as alkyl acrylates, alkyl thioacrylates, and N-vinyl lactams. The monomers are subsequently grafted to the pre-formed polymers via photochemical initiation.

U.S. patent 3,427,274 issued to Cornell describes hardenable materials formed from a mixture of methyl methacrylate homopolymer and styrene-butadiene copolymer latex coated with methyl methacrylate polymer which may be incorporated in a methacrylate-crosslinking agent composition to form hardenable compositions.

A dental restorative material is disclosed in U.S. patent 3,452,437 to Chang as being formed from the "diglycidyl methacrylate of bisphenol-A" (bis-GMA) to which a quantity of methyl methacrylate may be added. Inorganic fillers, such as glass fiber or glass beads, may be included to modify the resultant matrix.

U.S. patent 3,468,977 to Bruckmann et al teaches the formulation of dental compositions from a mixture of a polymer and a monomer. The pre-formed uncrosslinked polymer beads are allowed to swell with monomer which may contain a crosslinking agent. Acrylic materials may be used for both the monomer and the polymer.

Petner, in U.S. patent 3,470,615, teaches the formulation of a material suitable for use in the construction of dental appliances. A mixture of an uncrosslinked homopolymer and crosslinked copolymer is dissolved in a liquid polyglycol dimethacrylate to form a suspension which may be brushed on a substratum and subsequently hardened by heat to build up layers of polymeric material. A similar teaching may be found in U.S. patent 3,471,596, also to Petner et al. A thick liquid is provided which is useful in the building up of dental crowns and the like. The difunctional monomer may contain various thickening agents including poly(methyl methacrylate). In some embodiments, the poly(methyl methacrylate) may be supplemented with additional polymer which may be partially crosslinked with allyl metnacrylate.

U.S. patent 3,539,533 to Lee provides a filling material comprising a monomer solution filled with inorganic

particulate filler. The monomer solution may be a mixture of methacrylate monomers containing bisphenol-A dimethacrylate.

Polyfunctional methacrylate monomers, including "bisphenol-A glycidyl dimethacrylate" (bisGMA), may be polymerized together with an inorganic filler to yield dental compositions, as taught by U.S. patent 3,597,389 to Taylor. U.S. patent 3,629, 187 to Waller discloses the use of the isocyanate or diisocyanate adducts of bisphenol-A type compounds. These adducts are employed together with various inorganic fillers and liquid monomers to form liquid or paste compositions which are polymerizable either thermally or photochemically.

U.S. patent 3,647,498 to Dougherty discloses dental compositions which are composed of liquid-solid mixtures. The solid phase is an acrylate or methacrylate polymer in bead form, which polymer is not crosslinked. The liquid is a blend of polyglycol di-methacrylates and bisphenol-A dimethacrylate. Various modifications of bisphenol-A are disclosed as being especially useful. In U.S. patent 3,649,608 to Logemann, dental compositions are taught which comprise solid bead polymers or copolymers of methacrylate type materials. These uncrosslinked materials are swollen in a liquid acrylate type monomer to yield useful products.

U.S. patent 3,751,399 to Lee discloses improved compositions for dental use comprising aromatic and alicyclic polyacrylates which are mixed together with other polyacrylate compounds especially those containing bisphenol-A structures. Inorganic fillers are specifically disclosed as being useful additives ; pre-formed organic polymers are not used as fillers.

In U.S. patent 3,833,404 to Sperling, elastomers, especially acrylates, urethanes, butadienes, natural rubbers, and polyvinyl alcohol, are formulated which possess interpenetrating polymeric network type structures. These materials are disclosed as being "hard", but are used as vibration and sound damping insulators.

U.S. Patent N°. 3,961,379 to Highgate discloses an article manufactured from a crosslinked polymer which is swollen with a monomer containing a crosslinking agent. Thus, a replica of the finished article is swollen with the monomer solution which is then polymerized *in situ*. A finished, hard product is recovered. The material is not comprised of fungible materials, but rather is a unitary object entire and indivisible. This prior Specification does not disclose or suggest hardenable compositions comprising blends of monofunctional polymerizable monomers, discrete particles of crosslinked polymers and crosslinking agents for the monomers wherein the crosslinked polymer particles have been swollen by the monomer and the crosslinking agent, as in the present invention.

U.S. Patent N°. 3,534,122 to Cornell, discloses the production of a dental composition from a mixture of a particulate crosslinked polymer such as methyl methacrylate or ethylene glycol dimethacrylate with methacrylate ester/divinyl benzene monomer mixtures. This prior Specification does not disclose the blending of a monofunctional polymerizable monomer with a particulate crosslinked polymer where the crosslinked polymer particles are capable of being swollen by the monomer and wherein after blending the particles are allowed substantially fully to swell with the monomer and crosslinking agent.

According to the invention, a process for producing hard, water insensitive shaped dental appliance having a high grind resistance is privuded which comprises

(1) Forming a moldable composition by the steps of

(1a) blending a composition comprising

(A) 20 to 66% of liquid monofunctional polymerizable monomer,

(B) 7 to 27% of di- or polyfunctional crosslinking agent, which is reactive with said monomer,

(C) 10 to 70% of crosslinked polymer in the form of discrete particles having average diameters ranging from 0.001 µm, whereby at least 50% by weight of said particles have diameters less than 100 µm and being swellable by said monomer but insoluble therein, said crosslinked polymer having been obtained by polymerizing a mixture of monofunctional monomer and di- or polyfunctional crosslinking agent, whereby the amount of the crosslinking agent is chosen such that

(a) it is sufficient to prevent the particulate crosslinked polymer from losing its particulate discreteness upon exposure to the monomer compenent and

(b) it provides a crosslinked polymer with the capacity to swell with from 10 to 500% of its own weight of monomer component and

(c) it is within the range of 0.01% to 30% by weight of the crosslinked polymer and

(D) 0 to 50% of an uncrosslinked polymer capable of dissolving in component (A), said percentages being based on the total weight of A, B, C and D, whereby

(E) optionally one or more members selected from the group consisting of free radical initiators, photochemical initiators, activators, pigments, fillers, adhesion modifiers and radiopaquing agents is included ; and

(1b) aging said blend for a period of time sufficient to fully swell said crosslinked polymer particles with the mixture of said monomer and crosslinking agent dissolved therein, whereby optionnaly in a preswell stage crosslinked polymer particles are caused to swell with a mixture of polymerizable monomer and

crosslinking agent at a time preceeding the final mixing of the ultimate composition,

(2) thereafter shaping the obtained moldable composition and

(3) exposing said composition to heat or electromagnetic radiation to harden it and provide a shaped dental appliance.

It is critical that the crosslinked polymer particles be capable of being swollen by the monomer and that, after blending the particles be allowed fully to swell with the monomer and the crosslinking agent. The need for and importance of this swelling requirement is explained hereinafter. The steps described above result in the formation of interpenetrating polymeric networks as also referred to hereinafter. It is important to note that unless the aforesaid swelling is allowed to proceed, the benrfits of the present invention will not be attained and the fully interpenetrating polymeric networks will not be obtained.

G.B. Specification N°. 801824 discloses a means whereby aqueous suspension polymerisation forms globules of crosslinked polymer, such polymers being subsequently immersed in liquid monomer and used for the manufacture of artificial teeth.

G.B. Specification N°. 569975 also relates to tooth reconstructions employing resin-based materials and in one example described therein, a mixture of a co-polymer of methyl methacrylate and methallyl methacrylate may be combined with a mixture of methyl methacrylate monomer and methallyl methacrylate monomer in a given ratio. This Patent does not disclose or suggest that crosslinked polymers in the form of discrete particles having average diameters ranging from 0.001 µmm to 500 mm be blended with monomers and crosslinking agents and then allowed to swell until substantially fully swollen to result in a hardenable composition as in the present invention.

U.S. Specification N°. 3 914 341 is concerned with hydrophilic polymeric species capable of being swollen by aqueous fluids, and in particular discloses the addition of relatively highly crosslinked polymeric fillers in such hydrophilic systems ; such fillers being designed to be poorly swellable with wather. The compositions disclosed in this Patent are substantially different from those of the present invention, the filler particles thereof partially swelling with a monomeric mixture with the object of enforcing a strong chemical bond between the filler and the hydrogel matrix. The filler is added to the monomer mixtures in a non-swollen state, such that the monomer penetrates partially into the filler particles so that the bond is particularly good. This contrasts with the present invention where swelling of the crosslinked polymer particles is a paramount importance. Furthermore the prior method is directd to soft, water absorptive hydrogels rather than to materials for forming hard water impervious materials as described in connection with the present invention.

None of the foregoing patents discloses or suggests the process of the present invention.

Further aspects of the invention are defined in claims 2-5 appended hereto.

The process of this invention is useful for the formation, construction, and repair of dental appliances, artificail teeth and oral prosthetics.

The process of the present invention involve the formation of hardenable compositions which may easily and conveniently be molded by known techniques into prosthetic dental conventional prior art acrylic dental appliances. Notably, dental appliances such as, for example, prosthetic teeth produced in accordance with the invention are characterized by a grind resistance which is up to six times greater than the grind resistance of conventional plastic teeth commercially marketed at this time. Moreover, while conventional acrylic plastic teeth, upon grinding, tend to melt and curl yielding a soft plastic debris, teeth produced in accordance with the present invention yield fine, gritty debris upon grinding in generally the same fashion as do porcelain teeth.

Further, prosthetic teeth produced from the precursor blend compositions according to the process of the invention are characterized by a chemical resistance which far exceeds that of conventional acrylic plastic teeth and which approaches the chemical resistance of porcelain teeth. The solvent resistance of prosthetic teeth obtained according to the invention far surpasses that of commercially available acrylic teeth, as shown, for example, by the fact that prosthetic teeth produced according to the invention remain intact after three weeks of immersion in methyl methacrylate monomer, whereas conventional acrylic plastic teeth are structurally degraded by methyl methacrylate, usually in 24 hours or less.

Although possessing a superior chemical resistance, prosthetic teeth produced in accordance with the present invention have unexpectedly been found to establish an excellent chemical bond with commercial denture base systems using standard processing methods. Thus, teeth produced in accordance with the invention are superior to porcelain teeth in that they bond well to denture base, eliminating seepage between the tooth and denture base, thus avoiding foul odors and marginal staining.

In comparison to porcelain teeth, the prosthetic teeth porduced in accordance with the invention are characterized as fracture resistant during denture processing and impact resistant should the denture be accidentally dropped into a porcelain sink or to the floor. The teeth described herein also give no clicking sound when occluded against each other in denture as do porcelain teeth. In comparison with conventional acrylic teeth, the prosthetic teeth produced in accordance with the invention are characterized by outstanding monomer and

solvent resistance ; outstanding thermal stability, improved hardness, density, and stain resistance ; and excellent hydrolytic stability. Some precursor blend compositions of the invention also provide teeth which are inherently opalescent. This characteristic enhances the appearance of the teeth, making the teeth more "natural" in appearance than conventional acrylic plastic teeth. Finally, teeth produced according to the invention exhibit excellent gloss when molded. During denture fabrication the gloss of these teeth is maintained better than that of conventioanl plastic teeth, due to superior chemical resistance.

The prosthetic teeth thus formed may be further characterized as having a heterogeneous microstructure. Such microstructure, which is believed to be functionally related to the superior physical characteristics of the dental appliance formed in accordance with the practice of the invention, may be ascertained after proper preparation of a specimen of the articles through a suitable means of magnification.

If desired, a mixture of two or more different crosslinked polymers may be used. A characteristic of the crosslinked polymer is that it will be insoluble in, but will absorb or imbibe, the liquid polymerizable monomer component used in the preparation of the precursor blend. Uncrosslinked polymer, if used in addition to the crosslinked polymer, may be characterized as being capable of dissolving in or being dispersed by the liquid polymerizable monomer. The liquid polymerizable monomer component of the compositions of the invention is a monomer having the capacity to dissolve or disperse such uncrosslinded polymer, dissolve or become miscible with the crosslinking agent, and swell the particles of crosslinked polymer used in the practice of the invention. If desired, a mixture of two or more such liquid polymerizable monomers may be used.

It has been discovered that the relative proportions of the components of the precursor blend are critical to the attainment of the desired properties in the final hardened or cured product produced therefrom notably the grind resistance, wear resistance, bond strength, impact resistance, resistance to monomer and other solvents, stain resistance, thermal stability, and hydrolytic stability. Thus, it has been discovered that blends of from 10 to 70 weight percent of the crosslinked polymer, from 0 to 50 weight percent of the uncrosslinked polymer, from 20 to 66 weight percent of polymerizable monomer, and from 7 to 27 weight percent of crosslinking agent for said monomer, together with minor amounts of initiator and in some cases activator for the initiator, provide blends which are particularly useful in the production of prosthetic teeth and denture bases characterized by properties far superior to those of conventional acrylic systems now used in the art. Prosthetic teeth possessing outstanding grind resistance, wear resistance, resistance to monomer and other solvents, strain resistance, thermal stability, and hydrolytic stability may be produced in accordance with the present invention. The following ranges are preferred : 13 to 52 percent by weight of crosslinked polymer ; from 13 to 34 weight percent of uncrosslinked polymer ; from 25 to 55 percent by weight of polymerizable monomer ; from 7 to 22 percent by weight of crosslinking agent ; and up to 2 percent by weight of initiator.

The polymerizable monomer may be selected from the group consisting of methyl-, ethyl-, trifluoroethyl-, propyl-, butyl-, pentyl-, and hexyl-, acrylate and methacrylate.

The uncrosslinked polymer may be selected from the group consisting of polymerizd methyl-, ethyl-, propyl-, isopropyl- and butyl-acrylate and methacrylate and mixtures thereof.

In general, the crosslinked polymers which are useful in the practice of the invention are formed from monomers or blends of monomers together with crosslinking agents in proper proportion. The monomers suitable for use in the production of the crosslinked polymers useful in the practice of the invention, will generally comprise any of a wide variety of monomers such as, for example, acrylic and lower alkyl acrylic acid esters, N-vinyl lactams, acrylamides, acrylonitriles, styrenes, alkenes, and urethanes. Similarly, mixtures of two or more monomers may be employed to provide these crosslinked polymers.

Preferred monomeric species useful in the preparation of the crosslinked polymers to be used in the invention include acrylic and lower alkyl acrylic acid esters which generally conform to the structure :

(I)

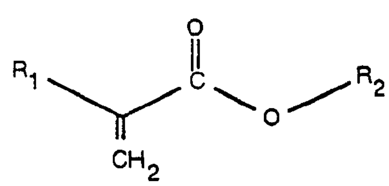

where $R_1$ is hydrogen or an alkyl group including from 1 to 6 carbon atoms, and where $R_2$ is either (a) an alkyl or cycloalkyl group including from 1 to 20, and preferably from 1 to 6 carbon atoms ; (b) phenyl ; and (c) alkyl subsituted phenyl in wich the alkyl groups include from 1 to 6 carbon atoms. Various substituents may be present on either o both of the groups $R_1$ and $R_2$. Thus, hydroxyl, amino, thiol and halogen (e.g., fluorine, chlorine, etc.) functionalities may be present, with the latter being preferred. Fluorine is an especially suuitable and useful substituent.

Especially preferred examples of monomers useful in the production of the crosslinked polymers used in the practice of the invention include methyl-, ethyl-, ispropyl-, tert-butyloctyl-, dodecyl-, cyclohexyl-, chloromethyl-, tetrachloroethyl-, perfluorooctyl-, hydroxyethyl-, hydroxypropyl-, hydroxybutyl-3-hydroxyphenyl-, 4-hydroxyphenyl-, aminoethyl-, aminophenyl-, and thiophenyl-, acrylate, methacrylate, ethacrylate, propacrylate, butacrylate and chloromethacrylate, as well as the homologous mono-acrylic acid esters of bisphenol-A, dihydroxydiphenyl sulfone, dihydroxydiphenyl ether, dihydroxybiphenyl, dihydroxydiphenyl sulfoxide, and 2,2 bis(4-hydroxy-2,3,5,6-tetrafluorophenyl)propane. Other suuitable species will be apparent to those skilled in the art. If desired, mixtures of two or more different monomers may be used to provide the crosslinked polymers useful in the practice of the invention.

The crosslinked agents which are useful in the production of the crosslinked polymer component to be used in the process of the invention comprise a wide variety of di- or polyfunctional moieties which are capable of crosslinking monomer species. In general, the reactive functionalities which serve as active sites for such crosslinking are ethylenic functions, but other reactive and effective crosslinking functions are similarly useful as will be hereinafter described. The use of crosslinking agents in the formulation of polymers is well known to those skilled in the art, who similarly recognize that it is necessary for such agents to have at least two reactive functionalities.

Suitable crosslinking agents may be selected from numerous families of polyfunctional monomers such as acrylic and lower alkyl acrylic acid diesters, acrylic and lower alkyl acrylic acid esters formed from alcohols, which alcohols have a second reactive function, urethane diacrylates and dimethacrylates, polyvinylic compounds, divinyl aromatic compounds and others, as will be apparent to those skilled in the art.

Preferably, the crosslinking agents comprise esters of unsaturated acids, e.g., acrylic, methacrylic, ethacrylic, propacrylic, butacrylic, etc., maleic, fumaric, citraconic, mesaconic, itaconic, malonic, or aconitic, etc., acids. Other unsaturated acids will be readily apparent to those skilled in the art. These acids are preferably reacted with either unsaturated or polyhydroxylic alcohols to form esters which are effective polyfunctional crosslinking agents useful in the formulation of the crosslinked polymers to be used in the process of the invention. In general, these alcohols have one or more hydroxylic functionality and have from 2 to 30 carbon atoms. Thus, useful alcohols include allyl, methallyl, crotyl, vinyl, butenyl, isobutenyl, and similar unsaturated alcohols as well as polyols such as ethylene glycol, propylene glycol, butylene glycol, diethylene glycol, triethylene glycol, tetraethylene glycol, pentaethylene glycol, glycerol, 1,3,3-trimethylolpropane, pentaerythritol, dihydroxyphenol, and alkylidene bisphenols such as bisphenol-A, 1,1,-bis(4-hydroxyphenyl)methane, 4,4'-dihydroxybiphenyl, 4,4'-dihydroxydiphenyl sulfone, dihydroxydiphenyl ether, dihydroxydiphenyl sulfoxide, resorcinol and hydroquinone.

Crosslinking agents preferred for the practice of the invention include the esters of a mono- or dibasic unsaturated acid with an unsaturated monohydroxylic alcohol such as allyl acrylate, allyl methacrylate, vinyl acrylate (methacrylate and $C_1$ to $C_{20}$ homologs), dimethallyl fumarate, N-allyl acrylamid, crotyl acrylate, allyl crotonate, allyl cinnamate and diallyl maleate. Other preferred species are the di-, tri-, and higher esters of polyhydroxylic alcohols such as ethylene "glycol" diacrylate (dimethacrylate and $C_2$-$C_{40}$ homologs), trimethylolpropane trimethacrylate, and the diacrylate and dimethacrylate esters of bisphenol-A as well as acrylate and alkyl acrylate esters which correspond to the general formula

(II)

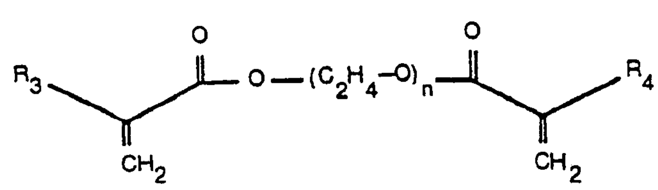

where $R_3$ and $R_4$ may be the same or different and are hydrogen or alkyl groups containing from 1 to 6 carbon atoms and n is a whole number from 1 to 10. Alternatively, the crosslinking agent may conform to the formula

(III)

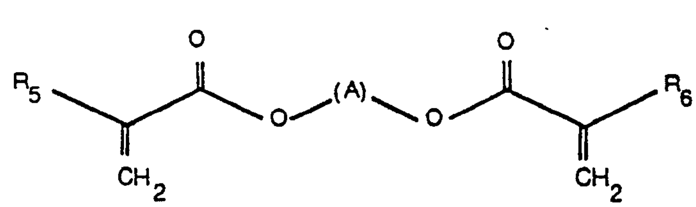

where $R_5$ and $R_6$ may be the same or different and are hydrogen or alkyl groups containing from 1 to 6 carbon

atoms and A is an aromatic moiety selected from the group consisting of

(a) biphenyl, diphenyl alkylidene having from 1 to 6 carbon atoms in the alkylidene portion thereof, diphenyl sulfone, diphenyl sulfoxide, diphenyl ether, and diphenyl sulfide ;

(b) the diglycidyl derivatives of group (a) ; and

(c) the diurethane derivatives of either group (a) or group (b). In addition, the crosslinking agent may be a glycidyl acrylate or allyl acrylate, divinyl (trivinyl or higher homologs) benzene, substituted divinyl benzenes, and analogous compounds. Furthermore, mixtures of two or more crosslinking agents are useful in the practice of the invention.

Compounds such as bis-GMA and the urethane diacrylate formed by reacting hydroxyethyl methacrylate with 2,2,4-trimethylhexyl-1,6-diisocyanate are especially useful, as are diallyl maleate, ethylene "glycol" dimethacrylate, trimethylolpropane trimethacrylate and the dimethacrylate ester of bisphenol-A.

The crosslinked polymers are produced by polymerizing a mixture of the monomer or monomers and crosslinking agent or agents described above. The amount of crosslinking agent employed in the production of the crosslinked polymers used in the practice of the invention is a critical factor. It has been found that the capacity of particles of polymers so produced to swell with or to imbibe the monomer component to form the precursor blend of the invention, is directly related to the amount of crosslinking agent used in the production of such crosslinked polymers.

The physiochemical properties of the crosslinked polymers useful in the practice of the invention determine the relative proportions of monomer and crosslinking agent used to formulate said suitable crosslinked polymers. Such crosslinked polymers must be sufficiently well crosslinked as to maintain substantially their structural identity when exposed to the monomer component of the precursor blend of the invention. At the same time, they must not be so thoroughly crosslinked as to be incapable of swelling with or imbibing the monomer component. Thus, it is convenient to describe the proportion of crosslinking agent by what it does rather than by what it is. In view of the fact that the crosslinked polymers are utilized in finely particulate form, as will be more fully explained, it is convenient to define the minimum amount of crosslinking agent used therein as being that amount which is sufficient to cause the particulate crosslinked polymer not to lose its particulate discreteness upon exposure to the monomer component of the invention. Similarly, the maximum amount of crosslinking agent used therein is that amount beyond which the resulting crosslinked polymer particles are unable to swell with or further imbibe a significant portion of monomer component upon exposure thereto. In this regard, a quantity of crosslinked polymer particles would be said to swell with or imbibe a significant portion of monomer component if it swelled with or has imbibed at least 10% of its own weight of monomer component. Preferably, an amount of crosslinking agent is used to provide a crosslinked polymer having the capacity to imbibe from 10 to 500 percent of its own weight of monomer component.

It will be clear to those skilled in the art that the minimum and maximum values for the proportions of crosslinking agents suitable for inclusion in the crosslinked polymers to be used in the process of this invention will vary depending upon the chemical identity of the component monomers and crosslinking agents. In general, however, the crosslinking agents may comprise from as low as 0.01% to as high as 30%, and preferably from 0.2% to 10% by weight of the resulting crosslinked polymer. For any monomer-crosslinking agent system, it is well within the routine knowledge of those skilled in the art to ascertain the optimum proportion of crosslinking agent in view of the requirements set forth above.

The production of the crosslinked polymers useful in the practice of this invention from monomers and crosslinking agents may be performed by any of the many processes known to those skilled in the art. Thus, the polymers may be formed by heating a mixture of the components to a temperature sufficient to cause polymerization, either with or without the addition of initiators. For this purpose, peroxy type initiators such as benzoyl peroxide, dicumyl peroxide and other materials familiar to those skilled in the art may be employed, and the use of activators may be advantageous in some formulations. Alternatively, the crosslinked polymers of the invention may be formed from the constituents by photochemical or radiant initiation utilizing light or high energy radiation. For photochemical initiation, photochemical sensitizers or energy transfer compounds may be employed to enhance the overall polymerization efficiency in manners well known to those skilled in the art.

The polymerization of the crosslinked polymers may be accomplished in a wide variety of ways, all of which are known to those skilled in the art. Thus, they may be formed by suspension polymerization as taught in U.S. patent 2,673,194 to Grim, emulsion polymerization, block polymerization or any other useful and convenient process. Since, as will be more fully described herein, it is desirable to have the crosslinked polymer available in the form of finely particulated granules or beads, suspension polymerization is especially convenient. Blocks of bulk-formed polymer may be crushed to yield a useful product, however. The size of the particles of crosslinked polymer is of significance to the invention. As indicated, it is desirable that the crosslinked polymer be in the form of small, discrete particles or beads. The average particle size should be from 0.001 micron to 500 µm. It is preferred that at least 50% by weight of the particles have diameters below 100 µm.

In addition to the crosslinked polymers described above, the polymer component of the precursor blend may comprise an uncrosslinked polymer. Such uncrosslinked polymer is formed from any of the monofunctional monomer species which have been disclosed above as being useful for the preparation of the crosslinked polymers used in the practice of the invention. Thus, monomer species conforming to Formula I above, the acrylic and $C_1$ to $C_6$ lower alkyl acrylicesters of aliphatic alcohols or phenols having from 1 to 20 carbon atoms, or mixtures thereof, are suitable as is vinylidene fluoride. Polymeric methyl methacrylate and methyl acrylate are preferred. While moieties conforming to Formula I above are most preferred, each and any of the other materials disclosed as being monofunctional monomers suitable for inclusion in the crosslinked polymer are also suitable materials for use in formulation of the uncrosslinked polymers. Mixtures of monomers are also quite useful. The uncrosslinked polymers may be formed from the monomers through any of the polymerization procedures known to those skilled in the art. Thus, thermal or photochemical polymerization, either with or without initiators, sensitizers, activators, or chain transfer agents, may be employed. Similarly, either bulk or suspension polymerization may be utilized. Preferably, the uncrosslinked polymers should be characterized as having average molecular weights of from about 100,000 to 2,000,000 g/mole, and especially of from about 500,000 to 900,000 g/mole. While the polymers are used in particulate form, they differ from the crosslinked polymers in that, unlike the crosslinked polymers, the uncrosslinked polymers do not have a critical particle size distribution. Thus, polymer particles or beads of any conveniently small size, such as 500 µm, may be utilized. Smaller sizes are preferred since they imbibe monomers and will dissolve therein more readily, but larger sizes may be used as well.

The uncrosslinked polymers used in the practice of the present invention are quite distinct from the crosslinked polymers. The crosslinked polymers have been defined as being capable of swelling with or imbibing the monomer component of the precursor blend of the invention, and as being of a physical and physiochemical structure so as not to lose their discrete particulate identity upon such swelling. This physical definition has, similarly, been related to the proportion of crosslinking agent included therein. By comparison, the particles of uncrosslinked polymer do not retain their particulate discreteness when exposed to the monomer component, but are dissolved therein if sufficient time and monomer component are provided.

The polymerizable monomers suitable for use in the process of the invention may comprise any of a wide variety of monomers. Thus, acrylic and lower alkyl acrylic acid esters, N-vinyl lactams, acrylamides, acrylonitriles, styrenes, alkenes, urethane acrylate or methacrylate, and other monomeric species may be employed in the practice of the invention.

Preferred monomeric species are acrylic and lower alkyl acrylic acid esters which may be seen generally to conform to Formula 1, above. Especially preferred examples of polymerizable monomers useful in the practice of the invention include methyl-, ethyl-, isopropyl-, t-butyl-, octyl-, dodecyl-, cyclohexyl-, chloromethyl-, tetrachloroethyl-, perfluorooctyl-, hydroxyethyl-, hydroxypropyl-, hydroxybutyl-, 3-hydroxyphenyl-, 4-hydroxyphenyl-, aminoethyl-, aminophenyl-, and thiophenyl-, acrylate, methacrylate, ethacrylate, propacrylate, butacrylate and chloromethacrylate, as well as the homologous mono-acrylic acid esters of bisphenol-A, dihydroxydiphenyl sulfone, dihydroxydiphenyl ether, dihydroxybiphenyl, dihydroxydiphenyl sulfoxide, and 2,2-bis(4-hydroxy-2,3,5,6-tetrafluorophenyl)propane. Other suitable species will be apparent to those skilled in the art who will further recognize that mixtures of two or more different polymerizable monomers may be used.

As noted above, the polymerizable monomer components of the precursor blends are generally liquid at room temperature and have the capacity to dissolve or disperse the uncrosslinked polymers and to swell or be imbibed by the crosslinked polymers which further comprise the precursor blend. Furthermore, the polymerizable monomers are capable of being crosslinked by the crosslinking agents as will be described below.

The crosslinking agents for the polymerizable monomers useful in the practice of the invention comprise a wide variety of di- or polyfunctional moieties which are capable of crosslinking monomeric species. In general, the reactive functionalities which serve as active sites for this crosslinking are ethylenic functions, but other reactive crosslinking functions are similarly useful. The use of crosslinking agents in the formulation and elaboration of polymers is well known to those skilled in the art, who will appreciate that it is necessary for such agents to have at least two reactive functionalities. Suitable crosslinking agents may be selected from numerous families of polyfunctional monomers such as acrylic and lower alkyl acrylic acid diesters, acrylic and lower alkyl acrylic acid esters formed from alcohols which alcohols have a second reactive function, urethane diacrylates and dimethacrylates, polyvinylic compounds, divinyl aromatic compounds and others as will be apparent to those skilled in the art.

Preferably, the crosslinking agents for the polymerizable monomers comprise esters of unsaturated acids, e.g., acrylic, methacrylic, ethacrylic, propacrylic, butacrylic, maleic, fumaric, citraconic, mesaconic, itaconic, malonic, or aconitic acids. Other unsaturated acids will be readily apparent to those skilled in the art. These acids are preferably reacted with either unsaturated or polyhydroxylic alcohols to form esters which are effective polyfunctional crosslinking agents for the monomeric species useful in the practice of the invention. Thus, useful

alcohols include allyl, methallyl, crotyl, vinyl, butenyl, isobutenyl and similar unsaturated alcohols as well as polyols such as ethylene glycol, propylene glycol, butylene glycol, diethylene glycol, triethylene glycol, tetraethylene glycol, pentaethylene glycol, glycerol, trimethylolpropane, pentaerythritol, dihydroxyphenol, alkylidene bisphenols such as bisphenol-A ; 1,1-bis(4-hydroxyphenyl)methane ; 4,4′-dihydroxybiphenyl ; 4,4′-dihydroxydiphenyl sulfone ; dihydroxydiphenyl ether ; dihydroxydiphenyl sulfoxide ; resorcinol and hydroquinone.

The preferred crosslinking agents used in the practice of the invention include the esters of a monomeric dibasic unsaturated acid with an unsaturated mono-hydroxylic alcohol such as allyl acrylate, allyl methacrylate, vinyl acrylate (methacrylate and homologs), dimethallyl fumarate, N-allyl acrylamide, crotyl acrylate, allyl crotonate, allyl cinnamate and diallyl maleate. Other preferred species are the di-, tri-, and higher esters of polyhydroxylic alcohols such as ethylene "glycol" diacrylate (dimethacrylate and $C_2$-$C_6$ homologs), trimethylolpropane trimethacrylate, and the dimethacrylate ester of bisphenol-A as well as other acrylate and alkyl acrylate esters corresponding to Formula II, above. Alternatively, the crosslinking agent may conform to Formula III, above. In addition, the crosslinking agent for the polymerizable monomers may be a glycidyl acrylate or allyl acrylate, divinyl (trivinyl or higher homologs) benzene, substituted divinyl benzenes, or analogous compounds. Furthermore, mixtures of crosslinking agents are useful in the practice of the invention.

Compounds such as bis-GMA and the urethane dimethacrylate formed from the reaction of hydroxyethyl methacrylate or acrylate with 2,2,4-trimethylhexyl-1,6-diisocyanate (hereinafter referred to as "urethane dimethacrylate" or "diacrylate" are especially useful, as are ethylene "glycol" dimethacrylate, trimethylolpropane trimethacrylate and the dimethacrylate ester of bisphenol-A. The corresponding acrylates are similarly useful as is diallyl maleate.

In addition to the components described above, (i.e., crosslinked polymer, uncrosslinked polymer, polymerizable monomer and a crosslinking agent for the polymerizable monomer) the precursor blend further may contain additional, optional, ingredients. These may comprise initiators, activators, pigments, fillers, radiopaquing agents, adhesion modifiers and other materials as will occur to those skilled in the art. Thus, it is useful to include free radical or photochemical initiators in the precursor blend composition to cause modification of the hardening kinetics thereof. In this regard, peroxy type initiators such as dicumyl or benzoyl peroxide are useful. Similarly, pigments and fillers may be added to modify the appearance, density, and physical characteristics of the resultant dental appliances. Inorganic materials, especially silica and titania, are useful fillers and pigments while a wide variety of other useful pigments and fillers will be apparent to those skilled in the art. While, in general, fillers and radiopaquing agents may constitute a major part by weight of the precursor blend composition, the initiators, activators, pigments, and adhesion modifiers should, taken as a whole, constitute a minor proportion by weight of the precursor blend compositions of which they are a part.

The precursor blends of the invention are formulated by a mixing together of the constituent species in proper proportion, followed by aging or maturing. Several techniques are available for this and others will be apparent to those skilled in the art. Thus, it is possible to combine crosslinked polymer, uncrosslinked polymer, polymerizable monomer and a crosslinking agent for said monomer in proper proportions including therewith, for example, a peroxide initiator and a pigment. This combination is then thoroughly mixed and aged to result in a precursor blend which has a uniform appearance. This blend may have the consistency of dough or may be more or less mobile depending upon the desired use therefor. The precursor blend thus formed may be alternatively molded, extruded, brushed, formed, worked or otherwise shaped in any conventional manner and caused to polymerize or cure to result in hard dental appliances having superior properties. The application of heat or radiant energy is usually required, for this polymerization or curing.

It is especially useful to mold precursor blends into artificial teeth for inclusion in prosthetic devices. It is to be understood, however, that the precursor blends are suitable for a very wide range of dental uses, including fillings, teeth, bridges, crowns, facings, pit and fissure sealants, denture base and denture reline materials, orthodontic splint materials, and adhesives for orthodontic appliances. The materials of the invention may also be utilized for prosthetic replacement or repair of various hard body structures such as bone and may be utilized for reconstructive purposes during surgery, especially oral surgery.

The nature of the chemical and physical relationships among the components of the precursor blends of the invention is important to the practice of the invention. Chief among these relationships is the necessity that the crosslinked polymer particles be capable of swelling with or imbibing the monomer component of the invention. Of similar importance is the requirement that the uncrosslinked polymers, when included, be capable of dissolving in the monomer component. In accordance with the invention, the precursor blend formed by any of the useful techniques described above is aged for a period of time sufficient to insure that the crosslinked polymer has become fully swollen with, interpenetrated by or has substantially imbibed the monomer-crosslinking agent mixture and that the uncrosslinked polymer, if used, has substantially dissolved therein. Thus, as used herein, "aged" or "aging" refers to the maintenance of the components of the precursor blend in association

with one another in the blend for a period of time sufficient to fully swell the crosslinked polymer particles with the mixture of polymerizable monomer and crosslinking agent dissolved therein. Frequently, the aging process is manifested by a change in the consistency of the mixture as equilibrium is approached. The time necessary to approach such equilibrium will vary depending upon the blending techniques, the relative proportions of materials, the particle sizes and molecular weights of the polymers and the temperature extant in the mixtures. In general, aging time of from one to seven days has been found to be adequate to approach the desired equilibrium. It is to be understood that it lies well within the abilities of those skilled in the art to ascertain the optimum aging time for a formulation in view of the foregoing considerations.

A further technique especially useful for the formulation of the precursor blends of the invention, denominated as the preswell method, causes the crosslinked polymer particles to swell with or imbibe a mixture of polymerizable monomer and crosslinking agent for said monomer at a time remote from and preceding the final mixing of the ultimate precursor blend. In accordance with this preferred technique, the crosslinked polymer particles are blended with a mixture of polymerizable monomer and crosslinking agent (dissolved in said monomer). The blend is then aged for a period of time sufficient to permit the crosslinked polymer particles to be fully swollen with, or interpenetrated by the monomercrosslinking agent mixture. In general, an amount of monomer is selected which will be completely imbibed by the crosslinked polymer particles with which the monomer is combined. This "preswollen" crosslinked polymer-monomer combination may subsequently be mixed with uncrosslinked polymer and further quantities of polymerizable monomer and crosslinking agent to form the precursor blend. This technique affords savings in time and results in greater convenience in the formulation of the precursor blends due to the fact that aging has taken place in advance of final mixing. Precursor blends thus formed may be alternatively molded, brushed, extruded,formed, worked or otherwise shaped in manners similar to those useful with batch mixing techniques to form similarly useful articles. Other techniques are presented in the examples which follow, and still others will be apparent to those skilled in the art.

Upon polymerization of the precursor blends, a three dimensional structure is believed to be formed which may be denominated as an interpenetrating polymeric network or IPN. The IPN structure which is thought thus to form is believed to be a major contributing factor to the serendipitous combination of superior chemical and physiochemical properties which is exhibited by the articles constructed according to the practice of the invention. Interpenetrating polymeric networks are related to, but distinct from, traditional graft polymers. In general, when a second polymer is synthesized in the intimate presence of a first polymer, the resultant material has been known as a graft polymer regardless of the actual extent of chemical grafting of one polymer to the other. IPN's are thought to be formed, however, when the first polymer is substantially crosslinked into a three dimensional network prior to the formation of the second polymer, and when that second polymer is caused to form in such a fashion that it too is substantially crosslinked into a three dimensional network.

Thus, an IPN may be viewed as being composed of two or more crosslinked, and hence three dimensionally arrayed, polymeric networks which co-exist in the same volume of space, but which do not necessarily have any covalent bonds in common. While the two networks may, indeed, by independent in the sense that they need posess no covalent linkages between them ; they are physically trapped one "within" the other and cannot disassociate by any physical manipulation without the rupture of covalent bonds.

Central to an understanding of interpenetrating polymeric networks is the recognition that an IPN is not a substance per se, but is, rather, a term descriptive of a structure. For discussions of the nature of IPN's in general, see the recent papers by L. H. Sperling et al, *Macromolecules*, vol. 9, N°. 4 (1976) ; *Macromolecules*, vol. 9, N°. 5 (1976) ; *J Polymer Science*, vol. 12, page 141 (1977) ; and *J Polymer Science*, vol. 16, page 583 (1978); and articles cited therein. Also, see Klepner et al, *J Elastoplast*, vol. 5, page 196 (Oct. 1973).

While it appears to be desirable that the crosslinking of both polymers be substantial, various degrees of crosslinking are possible in both the preformed polymer and the polymer formed *in situ*. In addition, it should be recognized that an IPN may be formed even when the initial and second polymers are formed from the same materials. For example, two independent networks of a polymethacrylate, suitably crosslinked, may interpenetrate each other to form an IPN. Similarly, an IPN need not be limited only to two networks, as mixtures of two or more polymers may be used as the initial polymer, and mixtures of two or more monomers may be employed to form a second polymeric network. Mixtures of two or more crosslinking agents may also be used in either network formation.

It is thought that in the present invention, interpenetrating polymeric networks may be formed. Thus, when particulate crosslinked polymer is allowed to swell with or imbibe monomer mixed with crosslinking agent, and when the imbibed mixture of monomer and crosslinking agent is subsequently caused to polymerize, an interpenetrating polymeric network may be seen to be formed within the confines of the particulate crosslinked polymer. It is believed that it is this interpenetrating polymeric network structure, which is localized in the particulate masses formed subsequent to the swelling of particulate crosslinked polymer and the polymerization of the precursor blend, that lends the superior chemical and physiochemical properties to the articles formed

according to this invention. It is believed that the aging process employed in the preparation of the precursor blends of the invention is required to accomplish substantially full swelling with, interpenetration by or substantially complete imbibition of monomer-crosslinking agent by the crosslinked polymer particles, and to approach an equilibrium thereof. It is to be understood, however, that the foregoing discussion of interpenetrating polymeric networks and their application to the present invention is not to be construed as a limiting factor thereof, but, rather, is to be interpreted as a mechanism which is proposed as being applicable in the present case.

As has been indicated, the compositions of the invention exhibit superior chemical and physiochemical properties. Accordingly, the articles made from the compositions of the invention exhibit superior grind resistance, monomer resistance, and bonding strength to denture bases. In addition, such articles display a unique microstructure.

Resistance to grinding may be demonstrated either qualitatively or quantitatively. Qualitatively, present plastic teeth tend to melt and curl when ground upon, whereas dental compositions disclosed herein exhibit a fine, dusty debris like that of porcelain teeth when ground upon in like manner using various speeds, torques, and hand pressures as follows :

1. *Low speed/high torque dental lathe* – Dedeco "Fast Tooth Grinding" wheel or Cratex 318-C wheel driven at 1740 or 3450 r.p.m. by a Red Wing dental lathe.
2. *Low speed/high torque dental handpiece* – An alundum or silicon carbide mounted point driven at 1400 r.p.m. by a belt-drive Foredom dental handpiece.
3. *Moderate speed/high torque dental handpiece* – An alundum or silicon carbide mounted point driven at 20,000 to 30,000 r.p.m. by a Dentsply Dentatus$_{tm}$ dental handpiece.
4. *High spee/low torque dental handpiece* – An alundum or silicon carbide mounted point driven at 250,000 to 350,000 r.p.m. by Dentsply Silencer$_{tm}$ dental handpiece.

Quantitative grind resistance is best measured through application of the test method detailed in the paper entitled *Quantitative Abrasive Grind Resistance Test* presented by F. Roemer, L. Tateosian and J Glenn at the 55th meeting of the American Association for Dental Research, 1977, Las Vegas, Nevada. Typical grind resistance values, depending on composition, are 90-140 g/s for present acrylic teeth ; 450-550 g/s for the dental compositions disclosed herein ; and 700/800 g/s for present porcelain teeth. Thus it is apparent that the present material is qualitatively and quantitatively superior to conventional plastic teeth in terms of grind resistance.

Porcelain teeth exhibit excellent methyl methacrylate (MMA) resistance ; they do not bond chemically to conventional denture base materials, however. The dental compositions disclosed herein exhibit excellent MMA resistance relative to present acrylic teeth. Immersed in MMA monomer at 23°C., present acrylic teeth will blanch, swell to one-and-one-half normal size or larger, crack, partially dissolve, and/or lose their original integrity within the first 24 hours of monomer contact. Teeth formed according to this invention do not exhibit any significant attack distortion after immersion in 23°C. MMA monomer for one week. Teeth formed from the preferred compositions have exhibited negligible attack after immersion in 23°C. MMA for 27 days.

The American Dental Association specification number 15 specifies, "the strength of the bond between tooth and resin is tested in tension. The minimum bond strength is 30.9 MN/m$^2$ (4,480 psi ; 315 kg/cm$^2$), which is sufficient to prevent separation of the teeth from the resin denture base in use." This pertains to "acrylic denture base resin polymerized by the heat processin technique." The compositions of this invention meet or exceed this specification.

A unique, heterogeneous microstructure is exhibited by appliances obtained according to this invention. One exemplary method for observing this microstructure is as follows :

1. The tooth, or molded article, is sectioned and one section potted in epoxy against a flat surface.
2. The sectioned surface of the potted specimen is polished to a smooth surface using nos. 320, 400 and 600 grit silicon carbide papers wet continuously with water.
3. A final polish is obtained using an aqueous slurry of 0.3 $\mu m$ $Al_2O_3$ on a chammy.
4. The polished surface of the section is exposed for four minutes to the vapors of boiling concentrated nitric acid ; the microstructure is oxidatively disclosed by this etchning procedure and is best captured by photomicrography at 260 X magnification.

The microstructure thus observed is heterogeneous and comprises what may best be described as particles suspended in a matrix. These particles are believed to be identifiable with the particulate crosslinked polymers of the precursor blend which have been swollen by and interpenetrated with the monomer and crosslinking agent. By comparison with conventional composite compositions containing only rigid inorganic fillers, the articles formed according to the present invention exhibit a microstructure in which the structure is much more closely packed ; It is to be understood that this methodology, while of wide application in the examination of the microstructure is not exclusive. Other techniques involving greater or lesser magnification and other means of visualization are also useful in disclosing the structure.

The following examples describe certain representative embodiments of this invention and will serve further to illustrate the nature thereof. It is to be understood that the examples are merely illustrative, and do not in any way limit the scope of the invention as defined by the claims. All percentages are by weight.

## Prosthetic Teeth

### Example 1

A precursor blend was prepared from the following composition :

| | |
|---|---|
| 47.83% | methyl methacrylate |
| 0.17% | benzoyl peroxide |
| 12.00% | 2,2-bis(4-methacryloxyphenyl)propane |
| 25.80% | poly(methyl methacrylate-co-ethylene dimethacrylate) (98.4:1.6) |
| 12.40% | poly(methyl methacrylate) |
| 1.80% | pigment |
| 100.00% | |

The crosslinked polymer was in the form of particles, 46% by weight of which were below 74 µm in sizes, the balance being below 500 µm in size. The poly(methyl methacrylate) had an average molecular weight of 800,000 g/mole.

The benzoyl peroxide and 2,2-bis(4-methacryloxyphenyl)propane were dissolved in the methyl methacrylate at ambient temperature to form a monomer solution. The polymers and pigment were charged to a planetary dough mixer containing the monomer solution and the charge was stirred until visibly homogeneous. Prosthetic teeth were molded from the resultant precursor blend mixture after it was aged at ambient temperature for seven days. The resulting teeth grind with a dusty, fine debris, bond to denture base and are impact and wear resistant.

### Example 2

The following composition yielded a precursor blend which could be molded into prosthetic teeth after processing according to the technique of Example 1

| | |
|---|---|
| 47.83% | methyl methacrylate |
| 0.17% | benzoyl peroxide |
| 12.00% | bis-GMA |
| 25.80% | poly(methyl methacrylate-co-ethylene dimethacrylate) (70:30) |
| 12.40% | poly(methyl methacrylate) |
| 1.80% | pigment |
| 100.00% | |

### Example 3

A two-step "preswell" mixing method was use to prepare a precursor blend from which prosthetic teeth were molded, said blend having the following composition :

| | | |
|---|---|---|
| Step 1 | 42.40% | methyl methacrylate |
| | 0.25% | benzoyl peroxide |
| | 6.00% | urethane diacrylate |
| | 1.50% | 2,2-bis(4-methacryloxyphenyl)propane |
| | 49.85% | poly(methyl methacrylate-co-ethylene dimethacrylate) (90:10) |
| | 100.00% | |

The crosslinked polymer was in the form of particles, 50% by weight of which were below 100 µm in size, the balance being below 500 µm in size.

| | | |
|---|---|---|
| Step 2 | 28.14% | poly(methyl methacrylate) |
| | 60.43% | methyl methacrylate |
| | 0.36% | benzoyl peroxide |
| | 10.20% | 2,2-bis(4-methacryloxyphenyl)propane |
| | 0.87% | pigment |
| | 100.00% | |

The poly(methyl methacrylate) had an average molecular weight of 850,000 g/mole.

The weight ratio of Step 1 to Step 2 material in this example is 1.14 to 1.00. Step 1 was achieved by preparing a solution of the monomers, crosslinkers and initiator and adding the crosslinked copolymer. This mixture was stirred for about two minutes to wet the polymer, capped against monomer loss, and held for one week at ambient temperature. The crosslinked copolymer completely absorbed the monomer solution during the one week "preswell" period. Although the copolymer was swollen by this process, the integrity of the individual copolymer particles was maintained. This "preswell" mixture was not gel-like, but had the consistency of a rubbery, spongy mass which was easily crumbled.

Step 2 was achieved by charging the "preswell", obtained in Step 1, to a planetary dough mixer and mixing sufficiently so as to break the "preswell" mass down to a fine consistency. The poly(methyl methacrylate) and pigment were added to the mixer and mixing was continued until a homogeneous dispersion was obtained. The solution of monomer and initiator, cited in the Step 2 composition, was charged to the mixer ; mixing continued until a homogeneous, gel consistency was obtained. The gellike mix was transferred to a holding container and aged at ambient temperature until a suitable consistency for molding prosthetic teeth was obtained, approximately three days.

Example 4

The two-step "preswell" method described in Example 3 was used to prepare a precursor blend from which prosthetic teeth were molded having the following composition :

| | | |
|---|---|---|
| Step 1 | 39.90% | methyl methacrylate |
| | 0.24% | benzoyl peroxide |
| | 9.98% | 2,2-bis(4-methacryloxyethoxyphenyl)-propane |
| | 49.88% | poly(methyl methacrylate-co-ethylene dimethacrylate) (98:2) |
| | 100.00% | |
| Step 2 | 36.37% | poly(methyl methacrylate) |
| | 49.92% | methyl methacrylate |
| | .32% | benzoyl peroxide |
| | 12.19% | 2,2-bis(4-methacryloxyethoxyphenyl)-propane |
| | 1.20% | pigment |
| | 100.00% | |

The weight ratio of Step 1 to Step 2 material in this example is 0.46 to 1.00. A suitable gel-like consistency for molding prosthetic teeth was obtained after aging at ambient temperature for 24 hours.

Example 5

A precursor blend was prepared from the following composition

| | |
|---|---|
| 24.67% | methyl methacrylate |
| 0.25% | benzoyl peroxide |
| 24.67% | ethylene 'glycol' dimethacrylate |
| 49.70% | poly(methyl methacrylate-co-ethylene dimethacrylate) (99.8:0.2) |
| 0.71% | pigment |
| 100.00% | |

The methyl methacrylate, benzoyl peroxide, and ethylene'glycol'dimethacrylate were mixed at ambient temperature to form a monomer solution. The polymer and pigment were charged to a planetary dough mixer containing the monomer solution and then mixed until visibly homogeneous. The polymer completely imbibed the monomer solution during the first seven days of contact at ambient temperature in a sealed container ; aging was continued for seven days prior to molding. Monolithic anterior prosthetic teeth were transfer molded by the following sequence :

1. 3 min. at 138°C., 290 psi ($20.4 \times 10^3$ g/cm$^2$).
2. 2 min. at 138°C., 1300 psi ($91.4 \times 10^3$ g/cm$^2$).
3. 5 min. cool at 1300 psi (91.4 0$\times 10^3$ g/cm$^2$).
4. 3 hr. at 118°C.

The resultant prosthetic teeth grind with a fine dusty debris, repolish to a high gloss, resist wear, resist methyl methacrylate and other solvents, are hydrolytically stable, show no visible degradation or distortion when heated at 220°C. for one hour, and bond well to denture base material.

One-Component Crown and Bridge Material

Example 6

The method described in Example 3 was used to prepare the following compositions :

| | | |
|---|---|---|
| Step 1 | 53.16% | methyl methacrylate |
| | 3.32% | ethylene 'glycol' dimethacrylate |
| | 9.97% | 2,2-bis(4-methacryloxyphenyl)propane |
| | 0.33% | benzoyl peroxide |
| | 33.22% | poly(methyl methacrylate-co-ethylene dimethacrylate) (95:5) |
| | 100.00% | |

The crosslinked polymer was in the form of particles, 50% by weight of which were below 100 µm in size, the balance being below 500 µm in size.

| | | |
|---|---|---|
| Step 2 | 37.00% | poly(methyl methacrylate) |
| | 49.43% | methyl methacrylate |
| | 12.36% | 2,2-bis(4-methacryloxyphenyl)propane |
| | 0.31% | benzoyl peroxide |
| | 0.09% | pigment |
| | 100.00% | |

The poly(methyl methacrylate) had an average molecular weight of 800,000 g/mole. The weight ratio of Step 1 to Step 2 material in this example is 1.50 to 1.00. This gel preparation was identical in utility to that described in Example 3.

The advantages of this material in crown and bridge restorations are that it evidences superior abrasion resistance over conventional acrylic crowns presently used ; it is a premixed moldable material which, unlike porcelain crown fabrication, does not require slow build-up techniques ; rudimentary, lowcost equipment may be used for its curing compared to high cost ovens necessary for porcelain crown fabrication ; and it has excellent polishability after laboratory fabrication and clinical fitting to proper occlusion.

One-Component Denture Base Material

Example 7

The gel composition, detailed in Example 6 and metod of preparation, described in Example 3 was used to prepare a denture base material. An upper denture containing commercial plastic teeth in the left quadrant and commercial porcelain teeth in the right quadrant was prepared using the gel, referred to above, for the denture base. The denture model was cast in wax, invested in stone, and the wax boiled out. The stone was coated with a separator fluid, and trial compression packed with the gel. The gel packed very well and additional packing was not required. The packed case was clamped and immersed in 71°C (160°F) water for 12 hours to effect polymerization. Replication of anatomical detail was excellent. The chemical bond to commercial plastic teeth and mechanical retention of commercial porcelain teeth was also excellent. The one-component gel prepared for use as a denture base material in this example was functional and practical after being stored at ambient temperature for 18 months.

## Claims

1. A process for producing a hard, water insensitive shaped dental appliance having a high grind resistance by

(1) forming a moldable composition by the steps of

(1a) blending a composition comprising

(A) 20 to 66% of liquid monofunctional polymerizable monomer,

(B) 7 to 27% of di- or polyfunctional crosslinking agent, which is reactive with said monomer,

(C) 10 to 70% of crosslinked polymer in the form of discrete particles having average diameters ranging from 0.001 μm to 500 μm, whereby at least 50% by weight of said particles have diameters less than 100 μm and being swellable by said monomer but insoluble therein, said crosslinked polymer having been obtained by polymerizing a mixture of monofunctional monomer and di- or polyfunctional crosslinking agent, whereby the amount of the crosslinking agent is chosen such that

(a) it is sufficient to prevent the particulate crosslinked polymer from losing its particulate discreteness upon exposure to the monomer component and

(b) it provides a crosslinked polymer with the capacity to swell with from 10 to 500% of its own weight of monomer component and

(c) it is within the range of 0.01% to 30% by weight of the crosslinked polymer and

(D) 0 to 50% of an uncrosslinked polymer capable of dissolving in component (A), said percentages being based on the total weight of A, B, C and D, whereby

(E) optionally one or more members selected from the group consisting of free radical initiators, photochemical initiators, activators, pigments, fillers, adhesion modifiers and radiopaquing agents is included ; and

(1b) aging said blend for a period of time sufficient to fully swell said crosslinked polymer particles with the mixture of said monomer and crosslinking agent dissolved therein, whereby optionally in a preswell stage crosslinked polymer particles are caused to swell with a mixture of polymerizable monomer and crosslinking agent at a time preceeding the final mixing of the ultimate composition,

(2) thereafter shaping the obtained moldable composition and

(3) exposing said composition to heat or electromagnetic radiation to harden it and provide a shaped dental appliance.

2. A process according to claim 1, wherein a crosslinked polymer with an amount of the crosslinking agent in the range of 0.2 to 30% by weight of the crosslinked polymer is used.

3. A process according to one of claims 1 or 2, wherein

(A) 25 to 66% of liquid monofunctional polymerizable monomer

(B) 7 to 22% of di- or polyfunctional crosslinking agent,

(C) 13 to 52% of crosslinked polymer and

(D) 13 to 34% of uncrosslinked polymer are blended.

4. A process according to one of claims 1 to 3, characterized in that the blend is aged for a period of time of 1 to 7 days.

5. A dental appliance, obtainable by the process of one of claims 1 to 4.

## Ansprüche

1. Verfahren zur Herstellung von harten, wasserunempfindlichen, geformten Dentalerzeugnissen mit hoher Abriebfestigkeit durch

(1) Bildung einer formbaren Zusammensetzung durch die Stufen Von

(1a) Mischen einer Zusammensetzung, umfassend

(A) 20 bis 66% flüssiges, monofunktionelles polymerisierbares Monomeres,

(B) 7 bis 27% di- oder polyfunktionelles Vernetzungsmittel, das mit dem Monomeren reaktiv ist,

(C) 10 bis 70% vernetztes Polymeres in der Form von diskreten Teilchen mit durchschnittlichen Durchmessern im Bereich von 0,001 µm bis 500 µm, wobei mindestens 50 Gew.-% der Teilchen Durchmesser von weniger als 100 µm aufweisen und in dem Monomeren quellbar sind, jedoch darin unlöslich sind, wobei das vernetzte Polymere erhalten wurde durch Polymerisation einer Mischung von monofunktionellem Monomeren und di- oder polyfunktionellem Vernetzungsmittel, wobei die Menge des Vernetzungsmittels derart gewählt wird, daß

(a) sie ausreicht, um zu verhindern, daß das teilchenförmige, vernetzte Polymere seine teilchenförmige Diskretheit verliert, wenn es der Monomerkomponente ausgesetzt wird, und

(b) sie ein vernetztes Polymeres schafft mit der Kapazität, mit von 10 bis 500% seines eigenen Gewichts an Monomerkomponente zu quellen, und

(c) sie im Bereich von 0,01 bis 30 Gew.-% der vernetzten Polymeren liegt und

(D) 0 bis 50% eines nicht vernetzten Polymeren mit der Fähigkeit, sich in Komponente (A) aufzulösen, wobei die Prozentangaben auf das Gesamtgewicht von A, B, C und D bezogen sind, wobei

(E) gegebenenfalls ein oder mehrere Mitglieder, gewählt aus der Gruppe, bestehend aus freien Radikalstartern, photochemischen Startern, Aktivatoren, Pigmenten, Füllstoffen, Ashäsionsmodifiziermitteln, und Radioopaque-Mittel, eingeschlossen sind ; und

(1b) die Mischung während einer Zeitspanne gealtert wird, die ausreicht, um die vernetzten Polymerteilchen vollständig zu quellen mit der Mischung des Monomeren und des darin aufgelösten Vernetzungsmittels, wobei man gegebenenfalls in einer Vor-Quellstufe vernetzte Polymerteilchen mit einer Mischung von polymerisierbarem Monomeren und Vernetzungsmittel quellen läßt, bevor das finale Vermischen der endgültigen Zusammensetzung erfolgt,

(2) anschließend die erhaltene formbare Zusammensetzung formt und

(3) die Zusammensetzung Hitze oder elektromagnetischer Bestrahlung aussetzt, um sie zu härten und einen geformten Dentalartikel zu schaffen.

2. Verfahren gemäß Anspruch 1, wobei ein vernetztes Polymeres mit einer Menge des Vernetzungsmittels im Bereich von 0,2 bis 30 Gew.-%, bezogen auf das vernetzte Polymere, verwendet wird.

3. Verfahren gemäß einem der Ansprüche 1 oder 2, wobei

(A) 25 bis 66% flüssiges monofunktionelles polymerisierbares Monomeres

(B) 7 bis 22% di- oder polyfunktionelles Vernetzungsmittel,

(C) 13 bis 52% vernetztes Polymeres und

(D) 13 bis 34% unvernetztes Polymeres vermischt werden.

4. Verfahren gemäß einem der Ansprüche 1 bis 3, dadurch gekennzeichnet, daß die Mischung während einer Zeitspanne von 1 bis 7 Tage gealtert wird.

5. Dentalartikel, erhältlich nach dem Verfahren von einem der Ansprüche 1 bis 4.

## Revendications

1. Procédé pour la production d'un appareil dentaire façonné, dur, insensible à l'eau, ayant une résistance élevée au broyage, procédé consistant

(1) à former une composition moulable, par les étapes consistant :

(1a) à mélanger une composition comprenant

(A) 20 à 66% d'un monomère polymérisable monofonctionnel liquide,

(B) 7 à 27% d'un agent de réticulation di- ou polyfonctionnel, réactif vis-à-vis de ce monomère,

(C) 10 à 70% d'un polymère réticulé se présentant sous forme de particules discrètes ayant un diamètre moyen compris entre 0,001 et 500 µm, au moins 50% en poids desdites particules ayant un diamètre inférieur à 100 µm et pouvant gonfler sous l'effet dudit monomère mais étant insolubles dans ce dernier, ledit polymère réticulé ayant été obtenu par la polymérisation d'un mélange d'un monomère monofonctionnel et d'un agent de réticulation di- ou polyfonctionnel, la quantité de l'agent de réticulation étant choisie de façon

(a) qu'elle soit suffisante pour empêcher que le polymère réticulé particulaire ne perde son caractère de particules discrètes après exposition au composant monomère, et

(b) qu'il donne un polymère réticulé ayant la possibilité de gonfler avec 10 à 500 % de son propre poids du composant monomère, et

(c) que cette quantité soit comprise entre 0,01 et 30% en poids du polymère réticulé, et

(D) 0 à 50% d'un polymère non réticulé pouvant se dissoudre dans le composant (A), lesdits pourcentages étant donnés par rapport au poids total de A, B, C et D, et, facultativement,

(E) un ou plusieurs composés choisis dans l'ensemble comprenant les amorceurs de radicaux libres, les amorceurs photochimiques, les activateurs, les pigments, les charges, les régulateurs d'adhérence et les agents opacifiants aux rayons X ; et

(1b) à vieillir ledit mélange pendant un laps de temps suffisant pour provoquer le gonflement complet desdites particules de polymère réticulé avec le mélange dudit monomère et de l'agent de réticulation qui y est dissous, auquel cas, facultativement, on provoque, dans un étage de prégonflement, le gonflement des particules de polymère réticulé avec un mélange d'un monomère polymérisable et d'un agent de réticulation à un instant qui précède le mélange final de la composition finale,

(2) puis à façonner la composition moulable obtenue, et

(3) à exposer ladite composition à la chaleur ou à un rayonnement électromagnétique pour le durcir et donner un appareil dentaire façonné.

2. Procédé selon la revendication 1, dans lequel on utilise un polymère réticulé avec une quantité d'agent de réticulation comprise entre 0,2 et 30% en poids par rapport au polymère réticulé.

3. Procédé selon l'une des revendications 1 ou 2, dans lequel on mélange

(A) 25 à 66% d'un monomère polymérisable monofonctionnel liquide,

(B) 7 à 22% d'un agent de réticulation di- ou polyfonctionnel,

(C) 13 à 52% d'un polymère réticulé et

(D) 13 à 34% d'un polymère non réticulé.

4. Procédé selon l'une des revendications 1 à 3, caractérisé en ce que le mélange est vieilli pendant 1 à 7 jours.

5. Appareil dentaire pouvant être obtenu par le procédé selon l'une des revendications 1 à 4.